(19) Europäisches Patentamt
European Patent Office
Office européen des brevets

(11) Veröffentlichungsnummer : **0 356 754 B1**

(12) # EUROPÄISCHE PATENTSCHRIFT

(45) Veröffentlichungstag der Patentschrift :
**04.03.92 Patentblatt 92/10**

(51) Int. Cl.$^5$ : **A61K 31/705, A61K 47/00**

(21) Anmeldenummer : **89114416.4**

(22) Anmeldetag : **04.08.89**

---

(54) **Transdermal anwendbare Arzneimittel mit einem Gehalt an pharmazeutisch einsetzbaren Glykosiden.**

---

(30) Priorität : **01.09.88 DE 3829640**

(43) Veröffentlichungstag der Anmeldung :
**07.03.90 Patentblatt 90/10**

(45) Bekanntmachung des Hinweises auf die
Patenterteilung :
**04.03.92 Patentblatt 92/10**

(84) Benannte Vertragsstaaten :
**AT BE CH ES FR GB IT LI LU NL SE**

(56) Entgegenhaltungen :
**DE-A- 2 759 171**
**FR-A- 2 380 030**
**US-A- 4 146 649**
**US-A- 4 333 926**

(73) Patentinhaber : **Roecar Holdings (Netherlands Antilles) N.V.**
**Kerkstraat 10a**
**Willemstad, Curacao Netherlands Antilles (NL)**

(72) Erfinder : **Walker, Hans Dr.**
**Hessenring 29**
**W-3440 Eschwege (DE)**
Erfinder : **Pegel, Karl Heinrich Dr.**
**King George V Avenue**
**Durban, Natal (ZA)**

(74) Vertreter : **Siewers, Gescha, Dr.**
**Rechtsanwälte Dr. Harmsen, Dr. Utescher**
**Dipl.-Chem. Harmsen, Bartholatus Dr.**
**Schaeffer, Dr. Fricke, Wolter Patentanwalt Dr.**
**Siewers Adenauerallee 28**
**W-2000 Hamburg 1 (DE)**

---

## Beschreibung

Die Erfindung betrifft neue transdermal anwendbare Arzneimittel mit einem Gehalt an pharmazeutisch anwendbaren Glykosiden.

Es gibt eine sehr große Anzahl wichtiger Arzneimittel, die als Glykoside vorliegen und in der Regel nur in dieser Form wirksam sind, während ihre Aglycone geringere oder andersartige Wirkungen aufweisen. Zu den vielfach verwendeten medizinisch wirksamen Glykosiden gehören beispielsweise die herzwirksamen Glykoside wie sie in Adonis-, Digitalis-, Nymphea-, Nerium-, Strophantus-, Convallaria-, oder Urgineaarten vorkommen. Es ist schon seit langem bekannt, daß solche glykosidischen Wirkstoffe häufig eine bessere oder zumindest ausgeglichenere Wirksamkeit dann aufweisen, wenn sie nicht als Reinsubstanzen, sondern in Form von pflanzlichen oder tierischen Extrakten verabreicht werden. Man hat daraus geschlossen, daß wahrscheinlich Begleitsubstanzen aus natürlichem Material als Schlepper wirksam sind, aber es ist sehr schwierig oder im Einzelfall bisher unmöglich, genau zu bestimmen, um welche Substanzen als Schlepper es sich hier handelt. Bei isolierten oder synthetisch hergestellten Glykosiden zeigt sich jedenfalls häufig, daß die Resorbierbarkeit im Vergleich zu Pflanzenextrakten geringer ist, so daß ein Teil dieser Glykoside nicht oder nur im geringem Umfang zur Therapie verwendet werden kann. Beispielsweise ist bekannt, daß bei peroraler Applikation Digitoxin zu 100%, Lanatosid A zu 70%, Lanatosid C zu nur 40%, g-Strophantin zu 2% und Gitoxin überhaupt nicht resorbiert werden. Man hat daher in gewissen Fällen auch schon versucht, die Resorption durch den Magen-Darmtrakt zu umgehen und die Wirkstoffe durch die Haut aufnehmen zu lassen In den Fällen, wo es sich bei den Wirkstoffen um Glykoside handelt, ist aber in der Regel die Resorption auch nur mangelhaft, während sie bei anderen Wirksubstanzen häufig recht gut ist. Aus der US-A-4 333 926 sind beispielsweise pharmazeutische Zubereitungen mit einem Glykosid als Wirkstoff und Cetylalkohol als Träger und aus der DE-A 27 59 171 sind Glykoside unter Zusatz von Cetylsterarylalkohol und dickflüssigen Paraffin als salbenförmige Zubereitung bekannt. Bei den zugesetzten Alkoholen handelt es sich allerdings um die üblicherweise zur Salbenherstellung benutzten Substanzen, also um freie Alkohole, die nicht weiter umgesetzt sind. Aus der FR-A 2380030 ist zwar die Verwendung von Glykosiden zusammen mit Propylenglykol bekannt, das in diesem Fall als alkoholisches Lösungsmittel verwendet wird. In der US-A-4146649 sind Mischungen aus Glykosiden und einem ethoxilierten Zucker, insbesondere ethoxilierte Glykose oder ethoxilierten höheren Alkoholen beschrieben. Diese Zubereitungen enthalten jedoch keine lipophilen Phasen.

Es wurde jetzt überrachend festgestellt, daß sich die Resorptivität von glykosidischen Wirkstoffen auf transdermalem Wege tatsächlich steigern läßt, wenn die Wirkstoffe zusammen mit Ethylenoxidaddukten mit etwa 20-30 Ethylenoxideinheiten an Sterole oder Alkohole mit etwa 12-30 C-Atomen verabreicht werden.

Es ist nicht vorhersehbar, daß es möglich ist, die nicht nur in Wasser, sondern in der Regel auch in lipophilen Lösungsmitteln schwerlöslichen Glykoside mit Hilfe von Lösungsvermittlern in eine Ölphase zu lösen und hieraus, und zwar auch dann wenn diese in Form einer Emulsion vorliegt, transdermal zur Resorption zu brigen, und zwar bei einer weitaus höheren Resorption als bisher jemals auf oraler oder transkutaner Route möglich war.

Erfindungsgemäß werden die Wirkstoffe zusammen mit ethoxilierten Sterolen oder ethoxilierten Alkoholen mit etwa 12-30 C-Atomen vermischt, und in mehrwertigen Alkoholen wie 1,2 - Propandiol oder Glycerin unter leichtem Erwärmen gelöst. Als Lösungsvermittler und Schlepper bei der späteren transdermalen Resorption können alle ethoxilierten Sterole verwendet werden, und zwar im Gemisch oder als Monoverbindung. Zu den geeigneten Sterolen gehören beispielsweise die Sitosterole, Cholesterol, Stigmasterol und die in der Natur vorkommenden Mischungen verschiedener Sterole, wie sie beispielsweise in Unverseifbaren von fetten Ölen gefunden werden. Als Alkohole mit etwa 12-30 C-Atomen können insbesondere Fettalkohole, aber auch natürlich vorkommende verzweigte, alicyclische oder aromatische Alkohole verwendet werden, wie z.B. Derivate des Squalens oder Phytol. Ein Teil dieser durch Umsetzen der freien Alkohole mit Ethylenoxid unschwer herstellbaren Verbindungen sind bereits die unter der Bezeichnung "Generol E" von der Firma Henkel KGaA vertriebene Produkte. Es handelt sich meist um Sitosterolgemische mit einem Ethoxilierungsgrad von 20-30. Bevorzugt eingesetzt wird ein ethoxiliertes Sitosterol mit einer Kettenlänge von 25 EO-Einheiten.

Die Lösungen der als Wirkstoffe eingesetzten Glykoside in Lösungvermittlern und mehrwertigen Alkohol lösen sich wiederum klar in Ölen oder geschmolzenen Fetten. Hier kommen vor allen Dingen die üblichen Salbengrundlagen in Frage wie beispielsweise mittelkettige Triglyceride, Glycerolmonostearat, Wollwachs, Schweineschmalz usw. Völlig überraschend hat sich auch herausgestellt, daß man nach dem Einarbeiten der Wirksubstanzen in die Fettgrundlagen diese zu Emulsionssalben weiterverarbeiten kann, da sich die Mischung ohne Schwierigkeiten mit etwa der gleichen Menge Wasser emulgieren läßt. Unabhängig davon, ob die Wirkstoffe in einer wasserfreien oder wasserhaltigen Fettgrundlage vorliegen, zeigen Untersuchungen mit $^{14}$C markiertem Wirkstoff, daß Penetration in die Haut und Permeation in etwa fünffacher Größenordnung erfolgen. Die Wirkstoffe dringen dabei nicht nur durch die Haut ein, sondern permeieren sogar durch die Haut hindurch, so daß sich bei großflächigerem Auftragen Plasmaspiegel in einer Höhe erreichen lassen, die auf intestinalem Wege nicht zu erzielen ist.

Die durchschnittliche Zusammensetzung für die transdermale Applikation beträgt etwa 0,01-0,1% Wirkstoff, 1% EO-Addukte und 10% mehrwertiger Alkohol. Nach dem Lösen des Wirkstoffes in der Mischung aus Addukten und mehrwertigem Alkohol wird die klare Lösung entweder mit der entsprechenden Menge wasserfreier Salbengrundlage vermischt oder in an und für sich bekannter Weise mit etwa der halben Menge Salbengrundlage vermischt, unter Zusatz der üblichen Menge nichtionogene Emulgatoren mit Wasser versetzt und in der üblichen Weise unter Rühren mit der Restmenge emulgiert.

Die Erfindung wird im folgenden anhand der Beispiele näher erläutert:

## Beispiel 1

Unter Erwärmen werden 100 mg Acetyldigoxin in 10,0 g ethoxiliertem Sojasterin (25 EO-Einheiten) und 100,0 g 1,2 Propandiol gelöst. Die Lösung wird auf etwa 70°C erwärmt und dann mit einer ebenfalls auf 70°C erwärmten Schmelze von 100,0 g Cetylpalmitat, 350 g Stearylheptanoat, 100 g Sorbitanmonostearat und 60,0 g PE-Sorbitanmonostearat gelöst. Unter intensivem Rühren wird mit destilliertem Wasser der gleichen Temperatur auf ein Gesamtgewicht von 1000,0 g aufgefüllt und abschließend die Emulsion kaltgerührt.

## Beispiel 2

In einer auf 70°C gehaltene Schmelze aus 300,0 g hydriertem Kokosöl, 50,0 g mittelkettiger Triglyceride, 120 g Glycerinmonostearat und 80,0 g ethoxiliertem Palmityl-Stearylalkohol (25 EO-Einheiten) wird eine Mischung aus 10,0 g ethoxiliertem Sojasterin (25 EO-Einheiten), 100,0 g 1,2 Propandiol und 20 mg K-Strophanthin eingerührt und diese Mischung anschließend mit auf 70°C gehaltenen Wasser zu 1000,0 g aufgefüllt. Die Emulsion wird dann langsam kaltgerührt.

## Beispiel 3

Penetration - bzw. Permeationsmessungen

Als Versuchstiere werden männliche, ca. 10 Wochen alte Kaninchen mit einem Körpergewicht von etwa 2 kg verwendet. Auf einer rasierten Fläche von 10 x 5 cm auf dem Rücken eines Kaninchens werden 1,0 g Creme mit einer Radioaktivität von 740 kBq (20 $\mu$ Ci) des jeweils ($^{14}$C-4) markiertem Glukosids aufgetragen, mit Alufolie abgedeckt und mit wasserfestem Heftpflaster fixiert. Nach 24 Stunden werden die Kaninchen eingeschläfert und der Applikationsbereich wird mehrmals mit ethanolfeuchten Tupfern gewaschen. Die Radioaktivität des Okklusivverbandsmaterials und der Tupfer wird nach Extraktion mit Ethanol gemessen. Die Haut des Anwendungsbereichs wird mit 2 m Natronlauge/Methanol/Triton® X 405 (6:3:1 v/v) bei 55 °C während 24 Stunden behandelt und danach mit 40°C warmen Ethanol gewaschen. Die Radioaktivität gemessen mit einem Flüssigkeits-Scintillationszähler (Philips PW 4700) ist ein Maß für die prozentuale Penetration der applizierten Substanzmenge. Die Permeation während 24 Stunden wird wie folgt berechnet: 100% - (Aktivität der Waschflüssigkeit + Aktivität der Penetrationsmenge) = % Permeation.

Bei Digoxin ergaben sich bei diesem Test Werte für die Penetration in Höhe von 2,1 % der applizierten Dosis und für die Permeation von 10,2 % der applizierten Dosis.

## Beispiel 4

Entsprechend Beispiel 3 wurde die Penetration bzw. Permeation von $^3$H-Ouabain gemessen.

Für Ouabain ergaben sich bei diesem Test Durchschnittswerte für die Penetration in Höhe von 1,1% der applizierten Dosis und für die Permeation von 55,4%. In Anbetracht der Tatsache, daß die Strophantine peroral nur sehr gering resorbiert werden, überrascht die hohe perkutane Aufnahme, die völlig neue Möglichkeiten für die medizinische Verwendung der Strophantine ergibt.

## Patentansprüche

1. Transdermal anwendbare pharmazeutische Zubereitungen mit einem Gehalt an Glykosiden als Wirkstoffe, gekennzeichnet durch die Verwendung ethoxilierter Sterole und/oder ethoxilierter Alkohole mit 12-30 C-Atomen als Lösungsvermittler in lipophiler Phase.

2. Pharmazeutische Zubereitung nach Anspruch 1, gekennzeichnet durch einen Gehalt an Sterolen und-/oder C12-C30 Alkoholen jeweils mit etwa 20-30 EO-Einheiten.

3. Pharmazeutische Zubereitung nach Anspruch 1 oder 2, gekennzeichnet durch einen Zusatz von Sitosterol mit etwa 25 EO-Einheiten.

4. Pharmazeutische Zubereitung nach Anspruch 1 bis 3, dadurch gekennzeichnet, daß die lipophile Phase als W/O-Emulsion vorliegt.

5. Pharmazeutische Zubereitungen nach Anspruch 1 bis 4, dadurch gekennzeichnet, daß als glykosidischer Wirkstoff herzwirksame Glykoside vorliegen.

## Claims

1. Transdermally applicable pharmaceutical compositions with a content of glycosides as active components, characterized by the use of ethoxylated sterols and/or ethoxylated alcohols with 12 - 30 C atoms as solubilizers in the lipophilic phase.

2. Pharmaceutical composition according to claim 1, characterized by a content of sterols and/or C12 - C30 alcohols with about 20 - 30 EO units each.

3. Pharmaceutical composition according to claim 1 or 2, characterized by the addition of sitosterol with about 25 EO units.

4. Pharmaceutical composition according to claim 1 - 3, characterized in that the lipophilic phase is present in the form of a water-in-oil emulsion.

5. Pharmaceutical composition according to claim 1 - 4, characterized in that it contains cardiotonic glycosides as glycosidic active components.

## Revendications

1. Compositions pharmaceutiques pour l'usage transcutané ayant une teneur en glycosides comme agents actifs, caractérisées par l'utilisation de stérols étholixiés et/ou d'alcools éthoxiliés avec 12 - 30 atomes C comme solubiliseurs dans la phase lipophile.

2. Composition pharmaceutique selon la revendication 1, caractérisée par une teneur en stérols et/ou en alcools C12 - C30 avec 20 - 30 unités EO environ respectivement.

3. Composition pharmaceutique selon la revendication 1 ou 2, caractérisée par l'ajoutage de sitostérol avec 25 unités EO environ.

4. Composition pharmaceutique selon les revendications 1 - 3, caractérisée par le fait que la phase lipophile se présente comme une émulsion type eau-en-huile.

5. Composition pharmaceutique selon les revendications 1 - 4, caractérisée par le fait qu'elle contient des glycosides cardiotoniques comme agents glycosidiques.